# EUROPÄISCHE PATENTSCHRIFT

(11) **EP 4 483 932 B1**
(45) Veröffentlichungstag und Bekanntmachung des Hinweises auf die Patenterteilung: **24.12.2025**
(21) Anmeldenummer: 24211884.2
(22) Anmeldetag: 26.08.2021
(51) Int. Cl.: A61M 16/00

(54) **BEATMUNGSVORRICHTUNG MIT AN EINER INNEREN FUNKTIONSANORDNUNG BEFESTIGTER EINGABE/AUSGABEVORRICHTUNG**
VENTILATION DEVICE COMPRISING AN INPUT/OUTPUT DEVICE SECURED TO AN INNER FUNCTIONAL ASSEMBLY
APPAREIL RESPIRATOIRE AVEC DISPOSITIF D'ENTRÉE/SORTIE FIXÉ À UN ENSEMBLE FONCTIONNEL INTERNE

(30) Priorität: 31.08.2020 DE 102020122672
(43) Veröffentlichungstag der Anmeldung: 01.01.2025
(62) Teilanmeldung aus: 21763387.4
(73) Patentinhaber: Hamilton Medical AG, 7402 Bonaduz (CH)
(72) Erfinder: Hepting, Daniel, 7026 Maladers (CH)
(74) Vertreter: Ruttensperger Lachnit Trossin Gomoll

(56) Entgegenhaltungen:
- US-A1- 2005 051 168
- US-A1- 2014 352 695
- US-A1- 2015 273 167
- US-A1- 2020 129 721

## Beschreibung

Die vorliegende Erfindung betrifft eine Beatmungsvorrichtung nach Anspruch 1 zur wenigstens unterstützenden künstlichen Beatmung von Patienten. Die Beatmungsvorrichtung umfasst:
- ein Vorrichtungsgehäuse,
- eine im Vorrichtungsgehäuse in einer Betriebsposition aufgenommene Funktionsanordnung, welche als Funktionseinheiten wenigstens einen Abschnitt einer Atemgasleitung, eine Druckveränderungsvorrichtung zur Veränderung eines Atemgasdrucks in der Atemgasleitung und eine Steuervorrichtung zur Steuerung des Betriebs wenigstens der Druckveränderungsvorrichtung aufweist, und
- eine am Vorrichtungsgehäuse in einer Anordnungsposition angeordnete, von außerhalb des Vorrichtungsgehäuses zu ihrer Bedienung zugängliche Eingabe/Ausgabevorrichtung zur Eingabe von Daten oder/und Steuerbefehlen an die Steuervorrichtung oder/und zur Ausgabe von Daten und Information, wobei die Eingabe/Ausgabevorrichtung durch eine Öffnung im Vorrichtungsgehäuse hindurch signalübertragungsmäßig mit der Steuervorrichtung verbunden ist.

Bevorzugt ist die Beatmungsvorrichtung eine Notfall-Beatmungsvorrichtung, wie sie von Notärzten und Ersthelfern in Notfallsituationen verwendet wird.

Als ein solches Notfall-Beatmungsgerät sind bekannt das Beatmungsgerät mit der Bezeichnung "EVE_{IN}" der Fritz Stephan GmbH in Gackenbach (DE) sowie das Beatmungsgerät mit der Bezeichnung "Falco 202 Evo" der italienischen Firma Siare Engineering International Group s. r. l. in Valsamoggia (IT).

Notfall-Beatmungsgeräte, unter anderem auch als "intensivmedizinische Beatmungsgeräte" bezeichnet, dienen der schnellen Versorgung eines Patienten mit Atemgas außerhalb einer klinischen Umgebung, also etwa an einem Unfallort oder/und während eines Transports eines Patienten. Selbstverständlich können Notfall-Beatmungsgeräte auch in einer klinischen Umgebung eingesetzt werden, jedoch stehen in Kliniken häufig leistungsfähigere Beatmungsgeräte als Notfall-Beatmungsgeräte zur Verfügung.

Als außerhalb einer klinischen Umgebung einsetzbare Beatmungsgeräte weisen Notfall-Beatmungsgeräte, wie auch bevorzugt die vorliegende Beatmungsvorrichtung, einen eigenen Energiespeicher auf, welcher zumindest für eine gewisse Dauer einen Betrieb des Notfall-Beatmungsgeräts unabhängig von einer Netzversorgung ermöglicht. Außerdem sind Notfall-Beatmungsgeräte hinsichtlich ihrer Größe und ihres Gewichts als tragbare Beatmungsgeräte ausgelegt, so dass sie von einem Notfall-Mediziner, etwa einen an einen Unfallort gerufenen Notarzt, nur mit eigener Muskelkraft auch über Strecken von mehreren Dutzend Metern ohne übermäßige körperliche Belastung bewegt werden können.

Sofern Notfall-Beatmungsgeräte ein Gebläse als eine bevorzugte Druckveränderungsvorrichtung aufweisen, können diese Notfall-Beatmungsgeräte ohne ergänzende Sondergasvorräte, wie etwa einen lösbar angekoppelten Sauerstoffvorrat, wenigstens Umgebungsluft als Atemgas verabreichen. Der Umgebungsluft kann bei Bedarf ein von der Umgebungsluft verschiedenes Sondergas, im häufigsten Fall reiner Sauerstoff, aber auch anästhetische oder/und therapeutische Gase und Gasgemische beigemischt werden. Hierzu weisen Notfall-Beatmungsgeräte, wie auch jenes der vorliegenden Erfindung, üblicherweise eine Anschlussformation zum Anschluss eines Sondergasvorrats auf.

Zum Schutz der Funktionsanordnung vor äußeren Einflüssen, wie mechanischen Stößen, Verschmutzung und dergleichen, ist diese im Vorrichtungsgehäuse angeordnet.

Damit die Eingabe/Ausgabevorrichtung eine Bedienung der Beatmungsvorrichtung sowie eine Wahrnehmung von Information über einen Betriebszustand der Beatmungsvorrichtung oder/und einen Beatmungszustand eines durch die Beatmungsvorrichtung beatmenden Patienten ermöglicht, ist diese am Vorrichtungsgehäuse angeordnet, und zwar derart, dass sie für eine Bedienperson von außerhalb des Vorrichtungsgehäuses zugänglich ist. Durch die signalübertragungsmäßige Verbindung von Eingabe/Ausgabevorrichtung und Steuerungsvorrichtung sind zwischen diesen Vorrichtungen Signale und somit Daten und Information übertragbar.

Aus der US 2015/0273167 A1 ist eine Beatmungsvorrichtung mit zwei miteinander durch Montage verbundenen Gehäusebauteilen bekannt. Eines dieser Gehäusebauteile ist in der US 2015/0273167 A1 als "blower box" bezeichnet und enthält ein Gebläse zur Förderung von Beatmungsgas. Eine im anderen Gehäusebauteil der bekannten Beatmungsvorrichtung aufgenommene und festgelegte Eingabe/Ausgabevorrichtung ist von außen durch eine Öffnung im Gehäusebauteil für ihre Bedienung durch eine Bedienperson zugänglich.

Aus der US 2020/0129721 A1 ist eine Beatmungsvorrichtung welche in gesonderten Gehäusen je eine Übertragungseinheit und eine Beatmungseinheit umfasst. Die gehäusemäßig eigenständigen Einheiten können bestimmungsgemäß körperlich und funktional zu der Beatmungsvorrichtung gekoppelt und wieder voneinander getrennt werden. Die Übertragungseinheit umfasst eine Eingabe/Ausgabevorrichtung zur Bedienung der Beatmungsvorrichtung sowie ein Exspirations- und ein Inspirationsventil, welche jeweils als Rückschlagventil ausgebildet sind. Die Beatmungseinheit umfasst eine Pneumatikeinheit, welche über eine interne Atemgasleitung Beatmungsgas zu einem Inspirations- und einem Exspirationsanschluss der Beatmungseinheit fördert. Die Beatmungseinheit umfasst weiter eine Steuerungseinheit zur Steuerung der Pneumatikeinheit. Eine Datenschnittstelle an der Beatmungseinheit können Signale zwischen der Steuerungseinheit und der an die Beatmungseinheit angekoppelte Übertragungseinheit übertragen werden.

Aus der US 2014/0352695 A1 ist eine über einen Touchscreen bedienbare Beatmungsvorrichtung bekannt, welche in ihrem Vorrichtungsgehäuse ein gesondertes, besonders geräuschemissionsgedämpftes inneres Gehäuse zur Aufnahme eines Gebläses der Beatmungsvorrichtung aufgenommen ist.

Aus der US 2005/0051168 A1 ist eine mit einer stationären Dockingstation verbindbare Beatmungsvorrichtung mit einem Roots-Gebläse bekannt.

Es ist Aufgabe der vorliegenden Erfindung, eine schnelle und sichere Montage der Beatmungsvorrichtung zu ermöglichen.

Diese Aufgabe wird erfindungsgemäß an einer eingangs genannten Beatmungsvorrichtung dadurch gelöst, dass die Eingabe/Ausgabevorrichtung unmittelbar an der Funktionsanordnung gegen ein Entfernen der Eingabe/Ausgabevorrichtung aus ihrer Anordnungsposition in einer Abheberichtung vom Vorrichtungsgehäuse weg gesichert ist. Somit kann eine umständliche Montage sowohl der Eingabe/Ausgabevorrichtung als auch Funktionsanordnung jeweils am Vorrichtungsgehäuse entfallen. Es kann dann ausreichen, nur eine Anordnung aus Funktionsanordnung und Eingabe/- Ausgabevorrichtung mit dem Vorrichtungsgehäuse zu verbinden. Die jeweils andere Anordnung wird dann von der einen Anordnung gehalten.

Grundsätzlich kann daran gedacht sein, die Eingabe/Ausgabevorrichtung auch am Vorrichtungsgehäuse gegen ein Entfernen in der Abheberichtung zu sichern, etwa durch Hintergreifen eines Randes einer Öffnung im Vorrichtungsgehäuse durch eine fest mit der Eingabe/Ausgabevorrichtung verbundene Formation, wie etwa einen Haken. Zur Vereinfachung der Montage - und bevorzugt auch der Demontage ist jedoch in einer bevorzugten Weiterbildung der Erfindung vorgesehen, dass die Eingabe/Ausgabevorrichtung nur an der Funktionsanordnung gegen ein Entfernen aus ihrer Anordnungsposition in der Abheberichtung vom Vorrichtungsgehäuse weg gesichert ist.

Grundsätzlich kann die Eingabe/Ausgabevorrichtung eine Öffnung des Vorrichtungsgehäuses durchsetzen, damit die Eingabe/Ausgabevorrichtung einerseits von außen zur Bedienung der Beatmungsvorrichtung zugänglich ist und damit andererseits die Eingabe/Ausgabevorrichtung mit der Steuervorrichtung signalübertragungsmäßig verbunden ist. Dabei ist es für ein möglichst dichtes Verschließen der von der Eingabe/Ausgabevorrichtung durchsetzten Öffnung des Vorrichtungsgehäuses vorteilhaft, wenn die Eingabe/Ausgabevorrichtung unter Zwischenanordnung eines Abschnitts des Vorrichtungsgehäuses zwischen einem Abschnitt der Eingabe/Ausgabevorrichtung und einem Abschnitt der Funktionsanordnung an der Funktionsanordnung gegen ein Entfernen aus ihrer Anordnungsposition in einer Abheberichtung vom Vorrichtungsgehäuse weg gesichert ist. Dann ist nicht nur die Eingabe/Ausgabevorrichtung an der Funktionsanordnung lagegesichert, sondern darüber hinaus können so die Eingabe/Ausgabevorrichtung und die Funktionsanordnung jeweils relativ zum Vorrichtungsgehäuse fixiert sein. Außerdem kann die Eingabe/Ausgabevorrichtung den zwischen der Eingabe/Ausgabevorrichtung und der Funktionsanordnung zwischenangeordneten Abschnitt des Vorrichtungsgehäuses außen überlappen. Der zwischenangeordnete Abschnitt des Vorrichtungsgehäuses, welcher in der Regel den Rand der von der Eingabe/Ausgabevorrichtung durchsetzten Öffnung des Vorrichtungsgehäuses umfasst, ist so sicher abgedeckt.

Eine von der Eingabe/Ausgabevorrichtung am zwischenangeordneten Abschnitt des Vorrichtungsgehäuses erzielte Dichtungswirkung kann noch dadurch erhöht werden, dass die Eingabe/Ausgabevorrichtung an der Funktionsanordnung in Richtung auf das Vorrichtungsgehäuse zu gespannt ist. Dadurch kann außerdem eine Klemmwirkung erzielt werden, mit welcher das Vorrichtungsgehäuse zwischen der Eingabe/Ausgabevorrichtung und der Funktionsanordnung geklemmt wird. Somit können die Eingabe/Ausgabevorrichtung, die Funktionsanordnung und das Vorrichtungsgehäuse als eine spielfreie Baueinheit nur durch Befestigung der Eingabe/Ausgabevorrichtung unmittelbar an der Funktionsanordnung gebildet werden.

Die Eingabe/Ausgabevorrichtung kann gemäß einer ersten Ausführungsform durch ein gesondert von der Eingabe/Ausgabevorrichtung und der Funktionsanordnung ausgebildetes Befestigungsmittel, wie etwa wenigstens eine Schraube oder/und wenigstens einen Clips, an der Funktionsanordnung gegen ein Entfernen aus ihrer Anordnungsposition in der Abheberichtung vom Vorrichtungsgehäuse weg gesichert sein.

Aus Gründen einer vorteilhaft kleinen Anzahl an Bauteilen, welche zur Bildung der Beatmungsvorrichtung benötigt werden, ist erfindungsgemäß vorgesehen, dass die Eingabe/Ausgabevorrichtung durch eine an der Eingabe/Ausgabevorrichtung ausgebildete oder angeordnete Verriegelungsformation und eine an der Funktionsanordnung ausgebildete oder angeordnete Verriegelungsgegenformation an der Funktionsanordnung gegen ein Entfernen aus ihrer Anordnungsposition in der Abheberichtung vom Vorrichtungsgehäuse weg gesichert ist. Die beiden Formationen: Verriegelungsformation und Verriegelungsgegenformation, sind dann vorteilhaft jeweils verliersicher an den sie tragenden Anordnungen: Eingabe/Ausgabevorrichtung und Funktionsanordnung, vorgesehen.

Bei betriebsbereiter Beatmungsvorrichtung steht die Verriegelungsformation mit der Verriegelungsgegenformation in einem Verriegelungs-Formschlusseingriff, welcher ein Entfernen der Eingabe/Ausgabevorrichtung aus ihrer Anordnungsposition in der Abheberichtung vom Vorrichtungsgehäuse weg verhindert. Erfindungsgemäß ist vorgesehen, dass wenigstens eine Formation aus Verriegelungsformation und Verriegelungsgegenformation dabei wenigstens einmal aus einer Freigabestellung längs einer Bewegungsbahn quer zu der Abheberichtung in eine Verriegelungsstellung beweglich ist. In der Freigabestellung besteht kein Verriegelungs-Formschlusseingriff zwischen Verriegelungsformation und Verriegelungsgegenformation. Die dann unverriegelte Eingabe/Ausgabevorrichtung ist dann aus ihrer Anordnungsposition in der Abheberichtung von dem Vorrichtungsgehäuse weg entfernbar. Im Gegensatz dazu ist in der Verriegelungsstellung der Verriegelungs-Formschlusseingriff hergestellt.

Durch die Bewegung der wenigstens einen beweglichen Formation aus Verriegelungsformation und Verriegelungsgegenformation von der Freigabestellung in die Verriegelungsstellung längs der Bewegungsbahn quer zur Abheberichtung kann der in der Verriegelungsstellung hergestellte Verriegelungs-Formschlusseingriff besonders hohe Kräfte in Abheberichtung abstützen, ohne dass dadurch der Verriegelungs-Formschlusseingriff wieder gelöst werden könnte. Bevorzugt ist daher die Bewegungsbahn der wenigstens einen beweglichen Formation aus Verriegelungsformation und Verriegelungsgegenformation orthogonal zur Abheberichtung orientiert. Denn eine längs der Abheberichtung wirkende Kraft hat keine orthogonal zur Abheberichtung verlaufende Kraftkomponente.

Grundsätzlich kann der Verriegelungs-Formschlusseingriff zwischen der Verriegelungsformation der Verriegelungsgegenformation durch einen Reibschluss zwischen einer Anlagefläche der Verriegelungsformation und einer bei hergestelltem Verriegelungs-Formschlusseingriff an der Anlagefläche anliegenden Gegenanlagefläche der Verriegelungsgegenformation gesichert sein. Der Reibschluss kann verhindern, dass sich die wenigstens eine bewegliche Formation aus der Verriegelungsstellung selbsttätig zurück in Richtung Freigabestellung bewegt oder durch im Betrieb und bei der Handhabung der Beatmungsvorrichtung auftretende Kräfte in Richtung Freigabestellung bewegt wird. Für eine stärker definierte Sicherung des Verriegelungs-Formschlusseingriffs kann die längs der Bewegungsbahn bewegliche Formation aus Verriegelungsformation und Verriegelungsgegenformation in der Verriegelungsstellung durch ein, optional mit Abstand von den aneinander anliegenden Flächen aus Anlagefläche und Gegenanlagefläche angeordnetes, Sicherungsmittel gegen eine Bewegung aus der Verriegelungsstellung heraus sicherbar sein.

Das Sicherungsmittel kann eine Verrastungsanordnung sein, welche eine Rastnase und eine Rastausnehmung oder/und eine Rastkante umfasst. Eine Rastformation aus Rastnase einerseits und Rastausnehmung oder/und Rastkante andererseits kann in Richtung eines Rasteingriffs federnd angeordnet sein, sodass sich ein Rasteingriff bei Erreichen einer vorbestimmten räumlichen Relativanordnung von Rastnase einerseits und Rastausnehmung oder/und Rastkante andererseits von alleine einstellen kann. Bevorzugt ist daher wenigstens eine der Rastformationen in ihre Rasteingriffsstellung vorgespannt. In der Rasteingriffsstellung taucht die Rastnase in die Rastausnehmung ein oder/und hintergreift die Rastkante. Die Rastkante kann eine die Rastausnehmung begrenzende Kante sein, sodass sowohl die Rastausnehmung als auch die Rastkante an ein und derselben Rastformation ausgebildet sein können.

Grundsätzlich kann die wenigstens eine bewegliche Formation aus Verriegelungsformation und Verriegelungsgegenformation unmittelbar, etwa durch eine Eingriffsöffnung im Vorrichtungsgehäuse, durch Handangriff von der Freigabestellung in die Verriegelungsstellung bewegbar sein. Zur Vermeidung unerwünschter Eingriffsöffnungen im Vorrichtungsgehäuse umfasst die Beatmungsvorrichtung bevorzugt ein Antriebsmittel, durch welches die bewegliche Formation von der Freigabestellung in die Verriegelungsstellung bewegbar ist.

Das Antriebsmittel kann ein Zug- oder/und Schubmittel sein, welches mit der beweglichen Formation zur Kraft- und Bewegungsübertragung verbunden ist. Bevorzugt ist das Zug- oder/und Schubmittel einstückig mit der beweglichen Formation verbunden, etwa als Spritzgussbauteil oder als sonst urformend hergestelltes Bauteil. Beispielsweise kann das Antriebsmittel eine Finger- oder Handangriffsformation sein, etwa ein Fingerring oder ein Zugbalken.

Für eine Übertragung hoher Verlagerungskräfte längs der Bewegungsbahn bei gleichzeitig hoher Verlagerungsgenauigkeit kann das Antriebsmittel einen selbsthemmenden Gewindetrieb umfassen. Aufgrund der selbsthemmenden Ausbildung des Gewindetriebs, was durch geeignete Wahl der Gewindesteigung realisierbar ist, kann der Gewindetrieb nicht nur das Antriebsmittel, sondern auch das Sicherungsmittel sein, welches die längs der Bewegungsbahn bewegliche Formation aus Verriegelungsformation und Verriegelungsgegenformation in der Verriegelungsstellung gegen eine Bewegung in die Freigabestellung sichert. Dann kann durch eine einzige Vorrichtung sowohl Antrieb als auch Positionssicherung der wenigstens einen beweglichen Formation erzielt werden, was die Anzahl an Bauteilen verringert, welche zur Bildung der Beatmungsvorrichtung erforderlich sind.

Der Gewindetrieb kann eine Gewindestange und ein relativ zur Gewindestange durch Drehung der Gewindestange um ihre Stangenlängsachse bewegliches und mit der Gewindestange in Schraubeingriff stehendes Bewegungsteil aufweisen. Das Bewegungsteil kann eine bewegliche Mutter, bewegliche Zahnstange oder bewegliches Zahnrad sein, wobei die Mutter, die Zahnstange oder das Zahnrad mit ihrer bzw. seiner Verzahnung in das Gewinde der Gewindestange eingreift. Die Gewindestange kann, wenngleich weniger bevorzugt, eine hohle Gewindestange mit Innengewinde sein, durch deren Drehung eine mit der hohlen Gewindestange in Schraubeingriff stehende Gewindestange mit Außengewinde längs der gemeinsamen Schraubachse verlagerbar ist. Bevorzugt weist jedoch die zur Drehung um ihre Stangenlängsachse betätigbare Gewindestange ein Außengewinde auf. Das Bewegungsteil ist mit der wenigstens einen längs der Bewegungsbahn beweglichen Formation aus Verriegelungsformation und Verriegelungsgegenformation zur gemeinsamen Bewegung gekoppelt. Die Gewindestange ist in der Regel um ihre Stangenlängsachse an einem Bauteil der Beatmungsvorrichtung drehbar gelagert und weist bezüglich dieses Bauteils nur diesen Rotationsfreiheitsgrad auf.

Grundsätzlich kann es ausreichen, wenn die längs der Bewegungsbahn bewegliche Formation aus Verriegelungsformation und Verriegelungsgegenformation nur ein einziges Mal bei der Herstellung der Beatmungsvorrichtung von der Freigabestellung in die Verriegelungsstellung bewegbar ist. Für eine wiederholte Wartung sowie erforderlichenfalls für eine Reparatur der Beatmungsvorrichtung ist es jedoch bevorzugt, wenn die längs der Bewegungsbahn bewegliche Formation reversibel zwischen Freigabestellung und Verriegelungsstellung bewegbar ist. Dann kann der Verriegelungs-Formschlusseingriff gelöst und erneut hergestellt werden.

Zur Klarstellung sei angemerkt, dass die Eingabe/Ausgabevorrichtung verriegelt oder unverriegelt in ihrer Anordnungsposition am Vorrichtungsgehäuse angeordnet sein kann, je nachdem, ob der Verriegelungs-Formschlusseingriff hergestellt ist oder nicht.

Das Antriebsmittel, insbesondere in seiner bevorzugten Ausgestaltung als selbsthemmender Gewindetrieb, weist bevorzugt eine Betätigungsformation auf, mittels welcher Antriebskraft, insbesondere als Antriebsdrehmoment, in das Antriebsmittel eingeleitet werden kann. Im Falle eines Gewindetriebs kann dies ein rotierbarer Körper sein, durch welchen von Hand oder durch Werkzeugeingriff ein Drehmoment in die Gewindestange einleitbar ist. Für einen Handangriff kann die Betätigungsformation eines Gewindetriebs ein gerändelter Zylinder, ein von einer Flügelschraube bekannter Flügelkopf oder allgemein ein rotationssymmetrischer Körper sein. Für einen Werkzeugeingriff kann die Betätigungsformation des Gewindetriebs eine bekannte Werkzeugeingriffsformation aufweisen, wie etwa ein Außen- oder ein Innensechskantprofil, einen Kreuzschlitz, einen einfachen Schlitz und dergleichen mehr.

Um die Betätigungsformation, welche nur zu Montage und Demontage der Beatmungsvorrichtung, insbesondere der Eingabe/Ausgabevorrichtung, betätigt werden muss, vor einer unbeabsichtigten Einleitung von Antriebskraft zu schützen, kann die Betätigungsformation des Antriebsmittels wenigstens in der Freigabestellung, vorzugsweise sowohl in der Freigabestellung als auch in der Verriegelungsstellung, der durch das Antriebsmittel längs der Bewegungsbahn bewegbaren Formation aus Verriegelungsformation und Verriegelungsgegenformation im Inneren des Vorrichtungsgehäuses angeordnet sein. Aus demselben Grunde ist bevorzugt das gesamte Antriebsmittel wenigstens in der Freigabestellung, vorzugsweise auch in der Verriegelungsstellung, im Inneren des Vorrichtungsgehäuses angeordnet. Außerdem kann dadurch das Antriebsmittel mit vorteilhaft kurzen Kraftübertragungswegen nahe bei der wenigstens einen beweglichen Formation angeordnet sein, da sich bei hergestelltem Verriegelungs-Formschlusseingriff bevorzugt beide Formationen aus Verriegelungsformation und Verriegelungsgegenformation im Inneren des Vorrichtungsgehäuses befinden.

Damit die Eingabe/Ausgabevorrichtung jene Öffnung des Vorrichtungsgehäuses, durch welche hindurch sie mit der Steuervorrichtung der Beatmungsvorrichtung signalübertragungsmäßig verbunden ist, in der Anordnungsposition vollständig verschließen kann, ist bevorzugt die wenigstens eine Betätigungsformation des Antriebsmittels, vorzugsweise das gesamte Antriebsmittel, durch eine andere Öffnung des Vorrichtungsgehäuses für eine Betätigung zugänglich ist als durch jene Öffnung, durch welche hindurch die Eingabe/Ausgabevorrichtung signalübertragungsmäßig mit der Steuervorrichtung verbunden ist.

Mit vorteilhaft kompakter Bauform weist das Vorrichtungsgehäuse bevorzugt eine prismatische Gestalt mit einer mit radialem Abstand von einer Prismenachse um die Prismenachse umlaufenden Gehäusemantelwand als einem Gehäusebauteil des Vorrichtungsgehäuses auf. Das Vorrichtungsgehäuse weist weiter wenigstens eine, bezogen auf die Prismenachse, axial endseitige Öffnung auf. Je nach Ausgestaltung des prismatischen Vorrichtungsgehäuses kann dieses nur eine axial endseitige Öffnung aufweisen, etwa wenn das Vorrichtungsgehäuse topfförmig mit einer sich an einem axialen Längsende orthogonal zur Prismenachse erstreckenden Stirnwand ausgebildet ist. Oder das prismatische Vorrichtungsgehäuse kann eine beidseitig axial offene Gehäusemantelwand aufweisen und dann zwei axial endseitige Öffnungen aufweisen, an jedem axialen Längsende je eine. Diese wenigstens eine axial endseitige Öffnung kann in einfacher Weise schnell und sicher durch einen Deckel verschlossen werden und bildet im unverschlossenen Zustand eine verhältnismäßig große Öffnungsfläche, welche eine Montage der Funktionsanordnung oder einzelner Funktionseinheiten im Inneren des Vorrichtungsgehäuses erleichtert. Wenigstens die Betätigungsformation des Antriebsmittels, vorzugsweise das gesamte Antriebsmittel, ist daher bevorzugt durch eine axial endseitige Öffnung der wenigstens einen axial endseitigen Öffnung für die Betätigung des Antriebsmittels zugänglich.

Das die Gehäusemantelwand aufweisende Gehäusebauteil ist bevorzugt ein rohrförmiges Gehäusebauteil, wobei dessen Rohrachse die Prismenachse ist. Das rohrförmige Gehäusebauteil ist aus Gründen geringen Gewichts und guter Wärmeleitung bevorzugt aus einem Leichtmetall, wie einer Aluminium- oder Magnesiumlegierung gebildet, kann zusätzlich oder alternativ auch aus einer Kupferlegierung gebildet sein, wie beispielsweise Messing oder Bronze, oder auch aus einer kupferhaltigen Legierung. Abweichend davon kann das rohrförmige Gehäusebauteil aus Kunststoff, insbesondere aus thermoplastischem Kunststoff, hergestellt sein. Zur Erhöhung der Wärmeleitfähigkeit kann der Kunststoff mit Partikeln gefüllt sein, welche die Wärmeleitfähigkeit der Mischung gegenüber der ungefüllten Kunststoffmatrix erhöhen. Ein solches Füllmaterial ist beispielsweise Bornitrid.

Aus Gründen erhöhter Stabilität ist das rohrförmige Gehäusebauteil bevorzugt fügestellenfrei gefertigt, etwa als Strangpressbauteil oder als Strangextrusionsbauteil.

Weiter bevorzugt weist das prismatische Gehäuse die Eingabe/Ausgabevorrichtung im Bereich seiner Gehäusemantelwand, vorzugsweise nur im Bereich seiner Gehäusemantelwand, auf.

In vorteilhafter Weise kann gemäß einer bevorzugten Weiterbildung der vorliegenden Erfindung die Eingabe/Ausgabevorrichtung spielfrei und sogar gegen Flüssigkeitseintritt dichtend am Vorrichtungsgehäuse angeordnet sein, wenn wenigstens eine Fläche, vorzugsweise beide Flächen, aus einer Anlagefläche der Verriegelungsformation und einer bei hergestelltem Verriegelungs-Formschlusseingriff an der Anlagefläche anliegenden Gegenanlagefläche der Verriegelungsgegenformation derart zur Bewegungsbahn geneigt sind, dass während einer Bewegung der wenigstens einen beweglichen Formation aus Verriegelungsformation und Verriegelungsgegenformation von der Freigabestellung in die Verriegelungsstellung zur Herstellung des Verriegelungs-Formschlusseingriffs durch einen gleitenden Anlageeingriff zwischen Anlagefläche und Gegenanlagefläche eine Kraftwirkung auf die Eingabe/Ausgabevorrichtung in Richtung auf das Vorrichtungsgehäuse zu ausgeübt wird. Bevorzugt sind wenigstens eine Fläche aus Anlagefläche und Gegenanlagefläche, vorzugsweise beide Flächen, sowohl zur Bewegungsbahn als auch zur Abheberichtung geneigt, wobei der Neigungswinkel relativ zur Abheberichtung wesentlich größer ist als relativ zur Bewegungsbahn, um durch Relativbewegung von Anlagefläche und Gegenanlagefläche längs der Bewegungsbahn eine hohe Kraft zwischen diesen Flächen parallel zur Abheberichtung zu erzeugen. Der oben erwähnte selbsthemmenden Gewindetrieb gestattet das bequeme Aufbringen der notwendigen hohen Verlagerungskräfte von Anlagefläche und Gegenanlagefläche längs der Bewegungsbahn zur Erzielung hoher auf die Eingabe/Ausgabevorrichtung parallel zur Abheberichtung wirkender Spannkräfte.

Wenngleich nicht ausgeschlossen sein soll, dass sowohl die Verriegelungsformation als auch die Verriegelungsgegenformation längs der Bewegungsbahn aufeinander zu und voneinander weg bewegbar angeordnet sind, ist es konstruktiv vorteilhaft und gestattet eine stabilere Ausbildung der Verriegelung der Eingabe/Ausgabevorrichtung am Vorrichtungsgehäuse, wenn nur eine Formation aus Verriegelungsformation und Verriegelungsgegenformation zwischen der Freigabestellung und der Verriegelungsstellung bewegbar ist. Bevorzugt ist dies die Verriegelungsgegenformation, sodass dann nur diese zwischen der Freigabestellung und der Verriegelungsstellung bewegbar ist. Im Vorrichtungsgehäuse besteht ausreichend Raum zur Aufnahme einer längs der Bewegungsbahn beweglichen Verriegelungsgegenformation, wohingegen die Eingabe/Ausgabevorrichtung in der Regel wesentlich kleiner als das Vorrichtungsgehäuse ist und somit die Ausbildung einer beweglichen Verriegelungsformation daran schwieriger ist als an der Verriegelungsgegenformation. Somit steht auch an der Funktionsanordnung mehr Bauraum zur Aufnahme des Sicherungsmittels oder/und des Antriebsmittels zur Verfügung als an der Eingabe/Ausgabevorrichtung.

Gemäß einer vorteilhaften Ausführungsform ist während einer Montage der Beatmungsvorrichtung die Funktionsanordnung durch eine Einführbewegung längs einer Einführbahn in ihre Betriebsposition im Vorrichtungsgehäuse einführbar. Bei Verwendung des oben genannten prismatischen Vorrichtungsgehäuses verläuft die Einführbahn wenigstens im Bereich einer endseitigen axialen Öffnung, durch welche hindurch die Funktionsanordnung bevorzugt in das Vorrichtungsgehäuse eingeführt wird, wenigstens tangential, vorzugsweise kollinear zur Prismenachse. Vorzugsweise ist die gesamte Einführbahn parallel oder kollinear mit der Prismenachse.

Die Eingabe/Ausgabevorrichtung ist durch eine Anordnungsbewegung längs einer Anordnungsbahn in ihre Anordnungsposition verlagerbar. Die Abheberichtung verläuft in der Regel parallel zur Anordnungsbahn.

Bevorzugt weist eine Anordnung aus der Eingabe/Ausgabevorrichtung und der Funktionsanordnung einen Stecker einer Signale oder/und Energie übertragenden Verbindungsleitung auf und die jeweils andere Anordnung aus der Eingabe/Ausgabevorrichtung und der Funktionsanordnung weist eine Buchse der Signale oder/und Energie übertragenden Verbindungsleitung auf, wobei der Stecker und die Buchse derart starr und längs der Einführbahn oder der Anordnungsbahn aufeinander zuweisend ausgerichtet mit der sie jeweils aufweisenden Anordnung verbunden sind, dass eine zeitliche Abfolge einer Montagebewegung aus Einführbewegung und Anordnungsbewegung und der jeweils anderen Montagebewegung aus Einführbewegung und Anordnungsbewegung eine funktionstüchtige Verbindung des Steckers mit der Buchse bewirkt. Somit kann eine gesonderte Herstellung der signalübertragungsmäßige Verbindung zwischen Steuervorrichtung und Eingabe/Ausgabevorrichtung entfallen, da diese signalübertragungsmäßige Verbindung mit der Positionierung der Funktionsanordnung im Vorrichtungsgehäuse sowie mit der Positionierung der Eingabe/Ausgabevorrichtung in ihrer Anordnungsposition selbsttätig hergestellt wird.

Die Eingabe/Ausgabevorrichtung umfasst bevorzugt wenigstens eine Anzeigevorrichtung, wie etwa einen Monitor, um damit Daten und Information für eine Bedienperson wahrnehmbar auszugeben. Bevorzugt umfasst die Anzeigevorrichtung einen Touchscreen, sodass sie auch zur Eingabe von Daten und Information dienen kann. Zusätzlich oder alternativ kann die Anzeigevorrichtung Leuchtmittel aufweisen, welche entweder erleuchtet oder nicht erleuchtet sein können und abhängig von ihrem Erleuchtungszustand Information ausgeben.

Zusätzlich oder alternativ kann die Eingabe/Ausgabevorrichtung wenigstens einen Schalter, wie etwa wenigstens einen Tastschalter oder/und wenigstens einen Drehschalter, als Eingabemittel zur Eingabe von Daten und Information aufweisen. Zur Erleichterung sowohl der Montage als auch der Bedienung der Beatmungsvorrichtung sind bevorzugt alle Eingabemittel, wie etwa Schalter, Touchscreen usw., der Beatmungsvorrichtung an der Eingabe/Ausgabevorrichtung angeordnet. Alternativ oder bevorzugt zusätzlich sind alle Ausgabemittel, wie Monitor, Leuchtmittel, Lautsprecher usw., der Beatmungsvorrichtung an der Eingabe/Ausgabevorrichtung angeordnet.

Grundsätzlich kann die Funktionsanordnung aus mehreren gesonderten Funktionseinheiten gebildet sein. Zur Erleichterung der Montage umfasst die Funktionsanordnung eine vormontierte Baugruppe aus wenigstens dem Abschnitt einer Atemgasleitung, der Druckveränderungsvorrichtung und der Steuervorrichtung, welche als vormontierte Baugruppe in das Vorrichtungsgehäuse einführbar oder/und aus diesem entnehmbar ist. Die vormontierte Baugruppe kann weitere Funktionseinheiten umfassen, wie etwa eine aktive oder passive Kühlvorrichtung zur Abführung von Wärme von der Druckveränderungsvorrichtung oder/und eine Aufnahme eines Energiespeichers oder/und den Energiespeicher selbst. Die Funktionsanordnung als vormontierte Baugruppe kann zusätzlich durch einzelne gesonderte Funktionseinheiten ergänzt sein, die nicht Teil der vormontierten Baugruppe sind.

Wegen der erleichterten Montage der Beatmungsvorrichtung umfasst die Funktionsanordnung bevorzugt alle Funktionseinheiten der Beatmungsvorrichtung als vormontierte Baugruppe, mit Ausnahme der Eingabe/Ausgabevorrichtung. Eine weitere Ausnahme kann ein elektrischer Energiespeicher sein, der austauschbar in einem Aufnahmeraum, etwa einem Schacht, aufgenommen sein kann. In diesem Fall kann die Funktionsanordnung als Teil der vormontierten Baugruppe bevorzugt den Aufnahmeraum zur Aufnahme eines elektrischen Energiespeichers aufweisen. Der elektrische Energiespeicher, etwa eine Batterie oder ein wiederaufladbarer Akkumulator kann bei der Montage der Beatmungsvorrichtung sogar in dem Aufnahmeraum lösbar aufgenommen sein. Alternativ kann in der Funktionsanordnung ein elektrischer Energiespeicher vormontiert verbaut sein. Dann kann auch der Energiespeicher Teil der vormontierten Baugruppe der Funktionsanordnung sein.

Ebenso kann ein Filter zur Reinigung von Atemgas Teil der Funktionsanordnung sein. Da der Filter in der Regel mit Fortdauer des Betriebs der Beatmungsvorrichtung verschmutzt und an Reinigungsleistung oder/und Volumendurchsatzkapazität einbüßt, ist er bevorzugt austauschbar an der Funktionsanordnung vorgesehen. Die vormontierte Baugruppe umfasst bevorzugt ein Aufnahmevolumen zur lösbaren Aufnahme eines Filters, insbesondere einer Filterkartusche. Eine Filterkartusche ist ein von Gas durchströmbares Gefäß mit einem darin aufgenommenen Filterkörper im Strömungsweg vom Gaseinlass zum Gasauslass des Gefäßes. So kann eine Handhabung des Filters erfolgen, ohne den Filterkörper als den Träger der Filter- und Reinigungsfunktion berühren zu müssen.

Ein Vorteil, wenn die Funktionsanordnung alle Funktionseinheiten der Beatmungsvorrichtung als vormontierte aufweist, liegt in der einfachen Wartung der Funktionseinheiten. Diese können als vormontierte Baugruppe nach einem Lösen des Verriegelungs-Formschlusseingriffs mit wenigen Handgriffen, besonders bevorzugt in einem einzigen Arbeitsschritt, durch Herausziehen aus dem Vorrichtungsgehäuse, insbesondere aus dem prismatischen oder rohrförmigen Gehäusebauteil, freigelegt und zugänglich gemacht werden. Als weitere Funktionseinheiten kann die Funktionsanordnung ein Netzteil, wenigstens ein Mischventil zur Mischung von Luft und wenigstens einem Sondergas, eine Kühlvorrichtung zur Abfuhr von Wärme aus der Umgebung des Gebläses, das oben genannte Antriebsmittel und das oben genannte Sicherungsmittel und dergleichen aufweisen. Die Kühlvorrichtung kann eine aktive Kühlvorrichtung sein, wie etwa ein Kühlgebläse, ist jedoch zur Reduzierung des Energieverbrauchs der Beatmungsvorrichtung bevorzugt eine passive Kühlvorrichtung, wie etwa ein Wärmeleitkörper aus gut wärmeleitfähigem Material, wie Metall, insbesondere Aluminium oder Kupfer oder Legierungen, die diese Metalle enthalten. Eine Wärmeübertragungsfläche der passiven Kühlvorrichtung kann zur Verbesserung des Wärmeübergangs von der Kühlvorrichtung zum Vorrichtungsgehäuse mit einer Spannvorrichtung gegen eine hierfür vorgesehene Anlagefläche des Vorrichtungsgehäuses gespannt sein. Die Spannvorrichtung kann eine oder mehrere Schrauben umfassen.

Das Vorrichtungsgehäuse, insbesondere das prismatische oder rohrförmige Gehäusebauteil, ist bevorzugt ein rein passives Bauteil und bildet eine Hülle um die Funktionsanordnung sowie eine Abstützung für die Eingabe/Ausgabevorrichtung.

Bevorzugt sind auch alle betriebsnotwendigen Anschlüsse, wie Buchsen, Gasleitungskupplungen, elektrische Anschlussleitungen, Gaseinlassöffnungen und Gasauslassöffnungen funktionsbereit an der Funktionsanordnung vorgesehen. Dann kann besonders vorteilhaft eine Endkontrolle der Funktionseinheiten auf ihr korrektes Funktionieren vor der Endmontage der Beatmungsvorrichtung durchgeführt werden, was den Aufwand zur Fehlerbeseitigung erheblich reduziert.

Bevorzugt umfasst die Druckveränderungsvorrichtung ein Gebläse. Sie kann zusätzlich oder alternativ wenigstens ein Ventil umfassen. Das Gebläse oder/und das Ventil sind durch die Steuervorrichtung in ihrem Betrieb steuerbar, d. h. wenigstens ein- und ausschaltbar. Bevorzugt ist das Gebläse durch die Steuervorrichtung in seiner Betriebsleistung veränderbar. Ebenso bevorzugt ist das wenigstens eine Ventil durch die Steuervorrichtung mit gestuft oder stufenlos veränderlichem Öffnungsquerschnitt betätigbar.

Die Eingabe/Ausgabevorrichtung ist bevorzugt unmittelbar durch den Verriegelungs-Formschlusseingriff an der Funktionsanordnung gesichert, wobei sich in der Regel ein Abschnitt des Vorrichtungsgehäuses zwischen einem Abschnitt der Funktionsanordnung einerseits und einem Abschnitt der Eingabe/Ausgabevorrichtung andererseits befindet, sodass die Eingabe/Ausgabevorrichtung durch den Verriegelungs-Formschlusseingriff mit der Funktionsanordnung mit Kraft gegen das Vorrichtungsgehäuse gespannt werden kann. Diese Ausbildung erfordert nicht nur eine geringe Anzahl an Bauteilen insgesamt, sondern verhindert auch eine Fehlmontage und eine dadurch notwendige Demontage mit erneuter Montage. Denn die Eingabe/Ausgabevorrichtung kann erst dann am Vorrichtungsgehäuse in der Anordnungsposition verriegelt werden, wenn sich die Funktionsanordnung korrekt in ihre Betriebsposition befindet, in welcher die Herstellung des Verriegelungs-Formschlusseingriffs möglich ist.

Bevorzugt sichert der Verriegelungs-Formschlusseingriff nicht nur die Eingabe/Ausgabevorrichtung gegen ein Entfernen vom Vorrichtungsgehäuse in Abheberichtung, sondern sichert auch die Funktionsanordnung gegen ein Entfernen vom Vorrichtungsgehäuse längs der Einführbahn in einer der Einführbewegung entgegengesetzten Richtung.

Dabei soll nicht ausgeschlossen sein, dass durch Herstellung des Verriegelungs-Formschlusseingriffs die Funktionsanordnung erst in ihre Betriebsposition gelangt. Jedenfalls kann bei der vorliegend diskutierten vorteilhaften Ausgestaltung der Beatmungsvorrichtung der Verriegelungs-Formschlusseingriff nur dann wirksam hergestellt sein, wenn sich die Funktionsanordnung in ihrer Betriebsposition und die Eingabe/Ausgabevorrichtung in ihrer Anordnungsposition befinden.

Für den Fall, dass das Vorrichtungsgehäuse eine prismatische Gestalt mit einer mit radialem Abstand von einer Prismenachse um die Prismenachse umlaufenden Gehäusemantelwand und mit wenigstens einer, bezogen auf die Prismenachse, axial endseitigen Öffnung aufweist, weist bevorzugt die Funktionsanordnung als Bauteil der vormontierten Baugruppe einen Deckel auf, welcher bei vollständig in die Betriebsposition eingeführter Funktionsanordnung eine axial endseitige Öffnung der wenigstens einen axial endseitigen Öffnung des Vorrichtungsgehäuses bedeckt. Der Deckel weist bevorzugt wenigstens einen betriebsnotwendigen Anschluss, vorzugsweise eine Mehrzahl von betriebsnotwendigen Anschlüsse auf, wie beispielsweise Buchsen oder/und Gasleitungskupplungen oder/und elektrische Anschlussleitungen oder/und eine Gaseinlassöffnung oder/und eine Gasauslassöffnungen.

Somit steht bis zur Anordnung der Funktionsanordnung in ihrer Betriebsposition eine axial endseitige Öffnung als Montageöffnung zur Verfügung, welche durch den Deckel der vormontierten Funktionsanordnung bei Anordnung der Funktionsanordnung in der Betriebsposition ohne weiteres verschlossen wird. Der Deckel dient somit auch zur visuellen Kontrolle der Anordnung der Funktionsanordnung in ihrer Betriebsposition im Inneren des Vorrichtungsgehäuses durch Betrachtung des Vorrichtungsgehäuses von außen.

Falls das Vorrichtungsgehäuse die oben genannte prismenförmige oder rohrförmige, beidseitig am Längsende offene Gehäusemantelwand und zusätzlich zum Deckel der vormontierten Baugruppe der Funktionsanordnung einen zweiten Deckel als Gehäusebauteil aufweist, wobei der zweite Deckel gesondert von der Funktionsanordnung an der Gehäusemantelwand als einem Gehäusebauteil des Vorrichtungsgehäuses zum Verschluss eines der Längsenden anbringbar und bevorzugt wieder abnehmbar ist, weist dieser zweite Deckel bevorzugt ihn durchsetzende Zugangsdurchgänge auf, durch welche hindurch wenigstens ein betriebsnotwendiger Anschluss, wie eine Buchse oder/und eine Gasleitungskupplung oder/und eine elektrische Anschlussleitung oder/und - besonders bevorzugt - eine Gaseinlassöffnung oder/und eine Gasauslassöffnung zugänglich ist.

Die vorliegende Erfindung wird nachfolgend anhand der beiliegenden Zeichnungen näher erläutert werden. Es stellt dar:
- Figur 1: eine perspektivische Ansicht einer erfindungsgemäßen Beatmungsvorrichtung in einer bevorzugten Ausführung als Notfall-Beatmungsgerät,
- Figur 2: eine perspektivische Explosionsansicht eines prismatischen Gehäusebauteils und der Funktionsanordnung des erfindungsgemäßen Notfall-Beatmungsgeräts von Figur 1,
- Figur 3: eine perspektivische Explosionsansicht der Funktionsanordnung und der Eingabe/Ausgabevorrichtung des erfindungsgemäßen Notfall-Beatmungsgeräts von Figur 1,
- Figur 4: Die Funktionsanordnung und die Eingabe/Ausgabevorrichtung von Figur 3 mit hergestelltem Verriegelungs-Formschlusseingriff, und
- Figur 5: eine Draufsicht auf die in Fig. 1 vom Betrachter abgewandte Stirnseite des Notfall-Beatmungsgeräts der Figur 1.

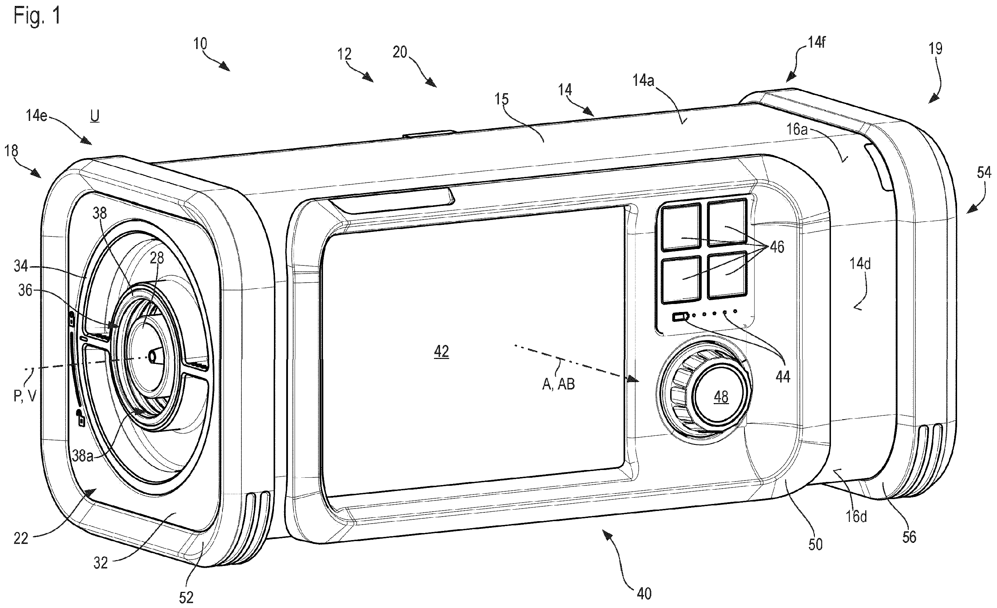

In Figur 1 ist eine erfindungsgemäße Ausführungsform einer erfindungsgemäßen Beatmungsvorrichtung in einer bevorzugten Ausführung als Notfall-Beatmungsgerät allgemein mit 10 bezeichnet. Das Notfall-Beatmungsgerät 10 umfasst ein Vorrichtungsgehäuse 12 mit prismatischer Grundform, vorliegend mit quaderartiger Grundform mit abgerundeten Kanten.

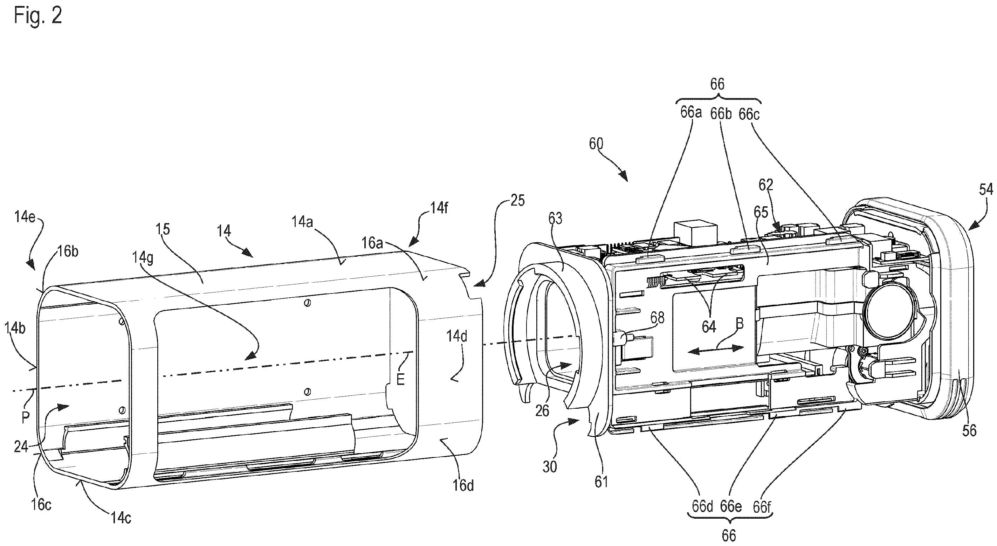

Die Gehäusemantelwand 14 des Vorrichtungsgehäuses 12 umfasst vier ebene Flächenanteile 14a, 14b, 14c und 14d (nur der obere und der vordere ebene Flächenanteil 14a bzw. 14d sind in Fig. 1 zu sehen, siehe ansonsten Fig. 2), von welchen jeweils in Umlaufrichtung um die Prismenachse P aufeinanderfolgende ebene Flächenanteile 14a, 14b, 14c und 14d zueinander orthogonal orientiert sind. Alle ebenen Flächenanteile 14a, 14b, 14c und 14d sind zur Prismenachse P parallel. Die ebenen Flächenanteile 14a, 14b, 14c und 14d sind durch gekrümmte, bevorzugt viertelzylindrische, Flächenanteile 16a, 16b, 16c und 16d (nur der vordere obere und der vordere untere gekrümmte Flächenanteil 16a bzw. 16d sind in Fig. 1 zu sehen, siehe ansonsten Fig. 2) miteinander bevorzugt fügestellenfrei verbunden. Die einzelnen Zylinderachsen der viertelzylindrischen und damit die gekrümmten Flächenanteile 16a, 16b, 16c und 16d sind parallel zur Prismenachse P. Bevorzugt ist die Gehäusemantelwand 14 durch ein extrudiertes Aluminiumrohr 15 als ein Bauteil des Vorrichtungsgehäuses 12 gebildet.

An der dem Betrachter von Figur 1 zugewandten Stirnseite 18 des Gehäuses 12 umfasst das Vorrichtungsgehäuse 12 einen vom übrigen Vorrichtungsgehäuse 20 längs der Prismenachse P abnehmbaren und am übrigen Vorrichtungsgehäuse 20 anordenbaren Gehäusedeckel 22. Der Gehäusedeckel 22 dient somit zum Verschließen einer Gehäuseöffnung 24 (siehe Fig. 2), welche an dem dem Betrachter von Figur 1 näher gelegenen Längsende 14e der Gehäusemantelwand 14 ausgebildet ist. Die Gehäuseöffnung 24 ist durch die Gehäusemantelwand 14 des Restgehäuses 20 begrenzt. Durch die Gehäuseöffnung 24 ist bei abgenommenem Deckel 22 ein Filter-Aufnahmefach 26 (s. Fig. 2) für eine Luftfilter-Kartusche 28 mit einem Luftfilter und ist ein Akku-Aufnahmefach 30 (s. Fig. 2) für einen wiederaufladbaren elektrischen Akkumulator als einen netzunabhängigen Energiespeicher zugänglich.

Der Gehäusedeckel 22 weist ein Deckelbauteil 32 und ein Verschlussbauteil 34 auf. Das Verschlussbauteil 34 ist am Deckelbauteil 32 um die Verschlussachse V drehbar gelagert. Die Verschlussachse V verläuft im Verschlusszustand, also dann, wenn der Gehäusedeckel 22 am Restgehäuse 20 angeordnet ist und die Gehäuseöffnung 24 verschließt, koaxial mit der Prismenachse P.

Der Gehäusedeckel 22 weist außerdem eine Umgebungsluft-Ansaugöffnung 36 auf, welche sowohl das Deckelbauteil 32 als auch das Verschlussbauteil 34 durchsetzt. Durch die Umgebungsluft-Ansaugöffnung 36 hindurch kann von einem Gebläse als einer Druckveränderungsvorrichtung Umgebungsluft aus der Umgebung U durch die Luftfilter-Kartusche 28 hindurch in einen Gebläseraum im Gehäuse 12 angesaugt werden.

Das Verschlussbauteil 34 ist in Figur 1 in seiner Verschlussstellung gezeigt, von welcher es entgegen dem Uhrzeigersinn etwa um eine Zwölftel-Umdrehung um die Verschlussachse V in eine durch ein Symbol in Gestalt eines offenen Vorhängeschlosses gekennzeichnete Offenstellung drehbar ist.

Die Umgebungsluft-Ansaugöffnung 36 ist nach radial außen - bezogen auf die Verriegelungsachse V - unmittelbar durch einen Lagerabschnitt 38 des Deckelbauteils 32 begrenzt. Der Lagerabschnitt 38 weist eine Befestigungsformation 38a in Form eines Innengewindes auf. An dieser Befestigungsformation 38a kann beispielsweise ein zusätzlicher Luftfilter angeordnet werden, welcher Filterfunktionen erfüllt, die der Luftfilter der Luftfilter-Kartusche 28 nicht leistet. Alternativ oder zusätzlich kann an der Befestigungsformation 38a eine Messvorrichtung angeordnet werden, welche die Umgebungsluft-Ansaugöffnung 36 durchströmende angesaugte Umgebungsluft messtechnisch erfasst, etwa deren chemische Zusammensetzung bestimmt oder bestimmt, ob und gegebenenfalls in welchem Ausmaß die angesaugte Umgebungsluft einen vorbestimmten Bestandteil enthält oder nicht.

Auf dem ebenen Flächenanteil 14d und ausgehend davon sich in die teilzylindrischen, in Umlaufrichtung um die Prismenachse P benachbarten Flächenanteile 16d und 16a erstreckend, weist das Notfall-Beatmungsgerät 10 eine in einer Anordnungsposition angeordnete Eingabe/Ausgabevorrichtung 40 auf, welche dem Informationsaustausch zwischen Bedienperson und Notfall-Beatmungsgerät 10 dient und welche der Steuerung des Notfall-Beatmungsgeräts 10 durch die Bedienperson dient. Die Eingabe/Ausgabevorrichtung 40 weist einen Monitor 42 als eine Ausgabevorrichtung auf, welcher vorzugsweise ein Touchscreen ist, der berührungssensitiv die Eingabe von Information gestattet. Die Eingabe/Ausgabevorrichtung 40 weist darüber hinaus Anzeige-LEDs 44 als weitere Ausgabevorrichtung beispielhaft zur Darstellung des Ladezustands des elektrischen Akkumulators des Notfall-Beatmungsgeräts 10 auf und weist beispielhaft Tastschalter 46 und einen Drehschalter 48 als Eingabemittel auf.

Zum Schutz vor äußeren Belastungen und zur Abdichtung gegenüber der Gehäusemantelwand 14 ist die Eingabe/Ausgabevorrichtung 40 durch einen Kunststoffrahmen 50, etwa aus thermoplastischem Kunststoff, umgeben. Im Rahmen 50 kann eine mit dem Rahmen 50 vollständig umlaufende elastomere Dichtung aufgenommen sein, welche sowohl am Rahmen 50, dort bevorzugt in einer Aufnahmenut, als auch an der Gehäusemantelwand 14 dichtend anliegt.

Der Gerätedeckel 22 ist durch einen in Umlaufrichtung vollständig um die Prismenachse P umlaufenden stoßdämpfenden Elastomerring 52 umgeben. Im Verschlusszustand bedeckt der Elastomerring 52 ebenso einen Teil der Gehäusemantelwand 14 wie der Stirnseite 18, sodass der Elastomerring 52 im Bereich des Gerätedeckels 22 das Notfall-Beatmungsgerät 10 sowohl gegen axiale als auch gegen radiale Stoßbelastungen schützt.

An dem dem Gerätedeckel 22 entgegengesetzten Längsende 14f der Gehäusemantelwand 14 ist ebenfalls ein Gerätedeckel 54 angeordnet. Im Gegensatz zum Gerätedeckel 22 ist der Gerätedeckel 54 jedoch bevorzugt nicht für sich alleine von der Gehäusemantelwand 14 des Gehäuses 12 abnehmbar. Um auch das Längsende des Gerätedeckels 54 vor axialen und radialen Stoßbelastungen zu schützen, ist auch an diesem Längsende ein vollständig geschlossen in Umlaufrichtung um die Prismenachse P umlaufender Elastomerring 56 vorgesehen, welcher sowohl einen Teil der Gehäusemantelwand 14 als auch einen Teil der Stirnseite 19 bedeckt. Die Stirnseite 19 ist der Stirnseite 18 bezüglich der Prismenachse P entgegengesetzt.

Im unverriegelten Zustand ist die Eingabe/Ausgabevorrichtung 40 längs einer Anordnungsbahn AB in einer Abheberichtung A aus der in Figur 1 gezeigten Anordnungsposition vom Vorrichtungsgehäuse 12 entfernbar.

In Figur 2 sind das prismatische Aluminiumrohr 15 als Bauteil des Vorrichtungsgehäuses 12 und eine im betriebsbereiten Zustand im Inneren des Vorrichtungsgehäuses 12 angeordnete Funktionsanordnung 60 in Explosionsansicht dargestellt.

Die Gehäusemantelwand 14 weist auf ihrer dem Betrachter von Figur 2 zugewandten Seite eine große Öffnung 14g auf, durch welche hindurch eine Steuervorrichtung 62 der Funktionsanordnung 60 mit der Eingabe/Ausgabevorrichtung 40 signalübertragungsmäßig verbindbar ist.

Die Funktionsanordnung 60 ist längs einer Einführbahn E, welche kollinear mit der Prismenachse P ist, durch eine axial endseitige Öffnung 25 am Längsende 14f der Gehäusemantelwand 14 bzw. des Aluminiumrohrs 15 in das Aluminiumrohr 15 als Teil des Vorrichtungsgehäuses 12 einführbar.

Die Funktionsanordnung 60 umfasst die oben bereits genannte Steuervorrichtung 62 an ihrer in Figur 2 nach oben weisenden Seite. Aus Gründen eines Entzündungsschutzes ist die Steuervorrichtung 62 baulich getrennt von inspiratorisches Atemgas, einschließlich reinen Sauerstoffs als optionales Sondergas, führenden Atemgasleitungen in der Funktionsanordnung 60 angeordnet. In einer Atmosphäre mit gegenüber normaler Luft erhöhtem Sauerstoffgehalt kann die Erwärmung elektrischer und elektronischer Komponenten der Steuervorrichtung 62 oder ein sich unerwünschterweise bildender Funke eine Zündwirkung haben, weshalb die ebenfalls vormontiert in der Funktionsanordnung 60 enthaltene Druckveränderungsvorrichtung in Gestalt eines Gebläses und die Atemgasleitungen, in welchen das von der Druckveränderungsvorrichtung geförderte Atemgas strömt, baulich strikt von der Steuervorrichtung 62 getrennt sind. Die Druckveränderungsvorrichtung und die Atemgasleitungen sind daher in Figur 2 nicht zu erkennen. Der Deckel 54 ist im dargestellten bevorzugten Ausführungsbeispiel ebenfalls Teil der als Baugruppe vormontierten Funktionsanordnung 60, die als eine Baugruppe in einem einzigen Montageschritt längs der Einführbahn E in das Aluminiumrohr 15 einführbar ist. Eine längs der Einführbahn E von einer Endplatte 61 der Funktionsanordnung 60 auskragende Eingriffsformation 63 dient der Anordnung des Deckels 22, insbesondere des Verschlussbauteils 34 am übrigen Vorrichtungsgehäuse 20 dann, wenn sich die Funktionsanordnung 60 in ihrer Betriebsposition im Inneren des Vorrichtungsgehäuses 12 befindet.

Zwei Platinenstecker 64 ragen von der Steuervorrichtung 62 parallel zur Anordnungsbahn AB hervor. Sie dienen der Herstellung einer signalübertragungsmäßigen Verbindung zwischen der Steuervorrichtung 62 und der Eingabe/Ausgabevorrichtung 40 unmittelbar alleine durch Anordnen der Funktionsanordnung 60 in ihrer Betriebsposition und durch Anordnen der Eingabe/Ausgabevorrichtung 40 in ihrer Anordnungsposition.

Dem Betrachter von Figur 2 zugewandt befindet sich an der Funktionsanordnung 60 längs einer Bewegungsbahn B parallel zur Einführbahn E bzw. zur Prismenachse P hin und her beweglich ein Träger 65 mit einer Verriegelungsgegenformation 66, welche sich in Figur 2 in ihrer Freigabestellung befindet, in der sie näher beim Deckel 54 angeordnet ist. Die Verriegelungsgegenformation 66 umfasst im dargestellten Beispiel sechs Verriegelungskeile 66a, 66b, 66c, 66d, 66e und 66f, von welchen drei an der Oberseite und drei an der Unterseite angeordnet sind. Alle Verriegelungskeile zusammen bilden die Verriegelungsgegenformation 66. Der Träger 65 ist relativ zur Endplatte 61 sowie zu den übrigen unbeweglichen Funktionseinheiten der Funktionsanordnung längs der Bewegungsbahn B beweglich.

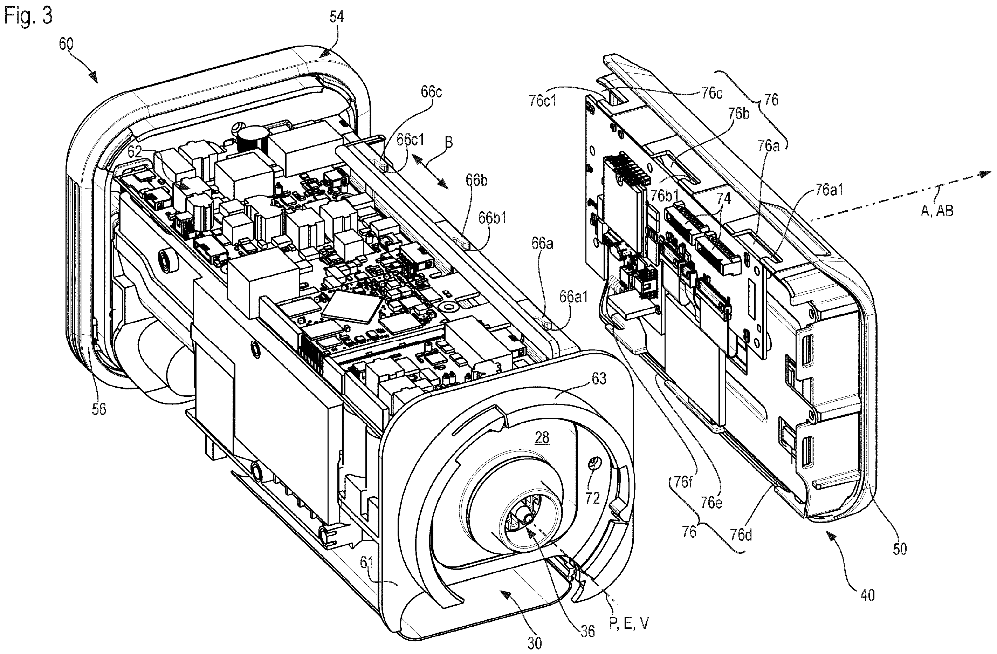

Der Träger 65, welcher beispielsweise als Kunststoff-Spritzgussbauteil ausgebildet ist, ist durch einen Gewindetrieb 68 längs der Bewegungsbahn B verlagerbar. In Figur 3 ist die Betätigungsformation 72 mit Kreuzschlitz-Werkzeugeingriffsformation einer Schraube bzw. Gewindestange des Gewindetriebs 68 rechts vom Filter-Aufnahmefach 26 dargestellt. Da der Gewindetrieb 68 selbsthemmend ist, kann nur über die Betätigungsformation 72 eine Bewegung des Trägers 65 bewirkt werden, jedoch keine Bewegung der Betätigungsformation 72 durch Bewegung des Trägers 65. Daher wirkt der Gewindetrieb 68 auch als Sicherungsmittel zur Sicherung der Position des Trägers 65 und damit der Verriegelungsgegenformation 66 in der jeweils durch Betätigung der Betätigungsformation 72 erreichten Stellung.

In Figur 3 ist die Eingabe/Ausgabevorrichtung 40 von schräg oben und hinten dargestellt, sodass von der Eingabe/Ausgabevorrichtung 40 hauptsächlich deren Rückseite erkennbar ist. Die Eingabe/Ausgabevorrichtung 40 umfasst elektronische Bauteile zum Betrieb des Monitors 42, der weiteren Anzeigevorrichtung 44 sowie der Schalter 46 und 48. Weiter umfasst die Eingabe/Ausgabevorrichtung 40 an ihrer Rückseite zwei parallel zur Anordnungsbahn AB, entgegen der Abheberichtung abstehende Verbindungsbuchsen 74, in welche die Platinenstecker 64 eingeführt werden, wenn sich die Funktionsanordnung 60 in ihrer Betriebsposition befindet und die Eingabe/Ausgabevorrichtung 40 entgegen der Abheberichtung A an die Gehäusemantelwand 14 angenähert wird.

Außerdem zeigt die Eingabe/Ausgabevorrichtung 40, vorzugsweise einstückig an ihrem Rahmen 50 ausgebildet, eine Verriegelungsformation 76, welche komplementär zur Verriegelungsgegenformation 66 der Funktionsanordnung 60 ausgebildet ist. Die Verriegelungsformation 76 weist insgesamt sechs Eingriffsausnehmungen 76a, 76b, 76c, 76d, 76e und 76f auf, in welche die Verriegelungskeile 66a, 66b, 66c, 66d, 66e und 66f zur Herstellung eines Verriegelungs-Formschlusseingriffs eingreifen. Im Gegensatz zur Verriegelungsgegenformation 66 ist die Verriegelungsformation 76 nicht relativ zu der sie tragenden Vorrichtung, hier: Eingabe/Ausgabevorrichtung 40, beweglich, sondern ist starr am Rahmen 50 der Eingabe/Ausgabevorrichtung 40 angeordnet.

Dann, wenn sich die Funktionsanordnung 60 in ihrer Betriebsposition befindet und sich die Verriegelungsgegenformation 66 in ihrer Freigabestellung befindet, kann die Eingabe/Ausgabevorrichtung 40 entgegen der Abheberichtung A in ihre Anordnungsposition verbracht werden, wobei die Verriegelungskeile 66a, 66b, 66c, 66d, 66e und 66f bereits in die Eingriffsausnehmungen 76a, 76b, 76c, 76d, 76e und 76f eingreifen, jedoch noch ohne einen ein Abheben der Eingabe/Ausgabevorrichtung 40 in der Abheberichtung A verhindernden Verriegelungs-Formschlusseingriff herzustellen. Dieser wird erst durch Bewegung der Verriegelungsgegenformation 66 längs der Bewegungsbahn B in Richtung vom Deckel 54 weg in die Verriegelungsstellung bewirkt.

Am Beispiel der Eingriffsausnehmung 76a ist in Figur 3 eine Anlagefläche 76a1 gezeigt, welche sich bei hergestelltem Verriegelungs-Formschlusseingriff in Anlage an einer Gegenanlagefläche 66a1 des Verriegelungskeils 66a befindet. Jede Eingriffsausnehmung 76a, 76b, 76c, 76d, 76e und 76f weist je eine Anlagefläche auf, wobei die Anlageflächen bevorzugt identisch sind. Der besseren Übersichtlichkeit halber sind in Figur 3 nur die oberen Anlageflächen 76a1, 76b1 und 76c1 mit Bezugszeichen versehen. Ebenso weist jeder Verriegelungskeil 66a, 66b, 66c, 66d, 66e und 66f je eine Gegenanlagefläche auf, wobei die Gegenanlageflächen bevorzugt identisch sind. Wiederum sind der besseren Übersichtlichkeit wegen nur die oberen Gegenanlageflächen 66a1, 66b1 und 66c1 mit Bezugszeichen versehen.

Die Anlageflächen und Gegenanlageflächen sind geringfügig bezüglich der Bewegungsbahn B geneigt, sodass sie bezüglich der Abheberichtung A geringfügig aus einer zur Abheberichtung A orthogonalen Bezugsebene geneigt sind. Die Neigung ist derart, dass bei Verlagerung der Verriegelungsgegenformation 66 längs der Bewegungsbahn von der Freigabestellung in die Verriegelungsstellung von den geneigten Gegenanlageflächen eine entgegengesetzt der Abheberichtung wirkende Kraft auf die Anlageflächen und damit auf die Eingabe/Ausgabevorrichtung 40 ausgeübt wird. Somit kann durch Herstellung des Verriegelungs-Formschlusseingriffs die Eingabe/Ausgabevorrichtung 40 durch die Verriegelungsgegenformation 66 zur Gehäusemantelwand 14 hin gespannt werden.

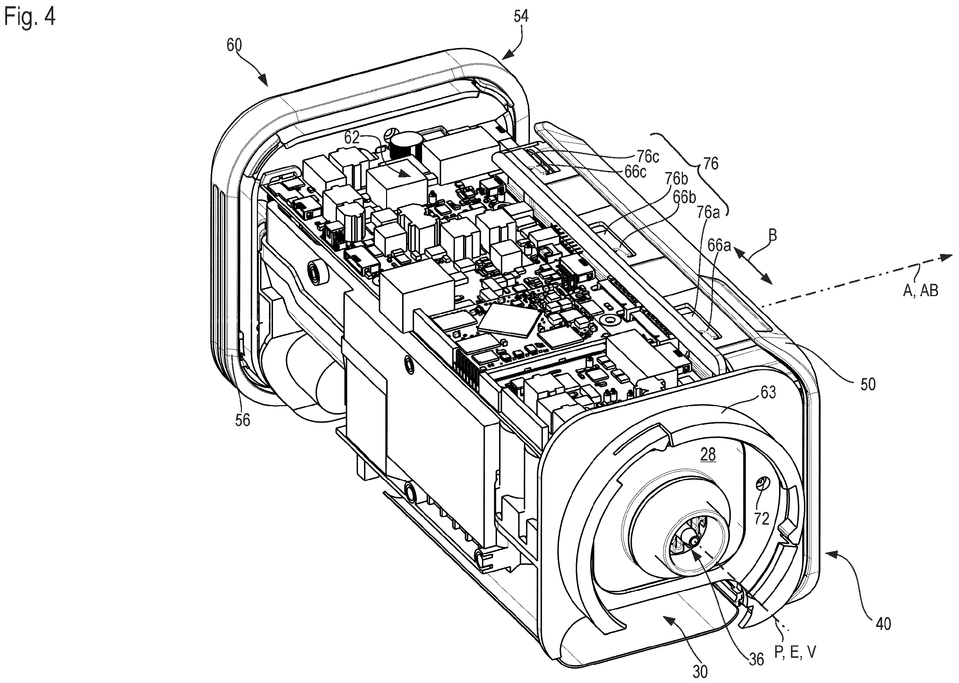

In Figur 4 sind die Funktionsanordnung 60 und die Eingabe/Ausgabevorrichtung 40 in der gleichen Perspektive wie in Figur 3 gezeigt, wobei die Eingabe/Ausgabevorrichtung 40 sich in ihrer Anordnungsposition befindet und die Verriegelungsgegenformation 66 in ihre Verriegelungsstellung verbracht ist. Somit ist die Eingabe/Ausgabevorrichtung 40 zum einen durch Einführen der Platinenstecker 64 in die Buchsen 74 signalübertragungsmäßig mit der Steuervorrichtung 62 verbunden und zum anderen durch Herstellung des Verriegelungs-Formschlusseingriffs zwischen der Verriegelungsformation 76 und der Verriegelungsgegenformation 66 gegen ein Entfernen in Abheberichtung A von der Funktionsanordnung 60 und von der in Figur 4 nicht dargestellten Gehäusemantelwand 14 gesichert. Der Gewindetrieb 68 hält mit seiner selbsthemmenden Wirkung die Verriegelungsgegenformation 66 in der in Figur 4 gezeigten Verriegelungsstellung.

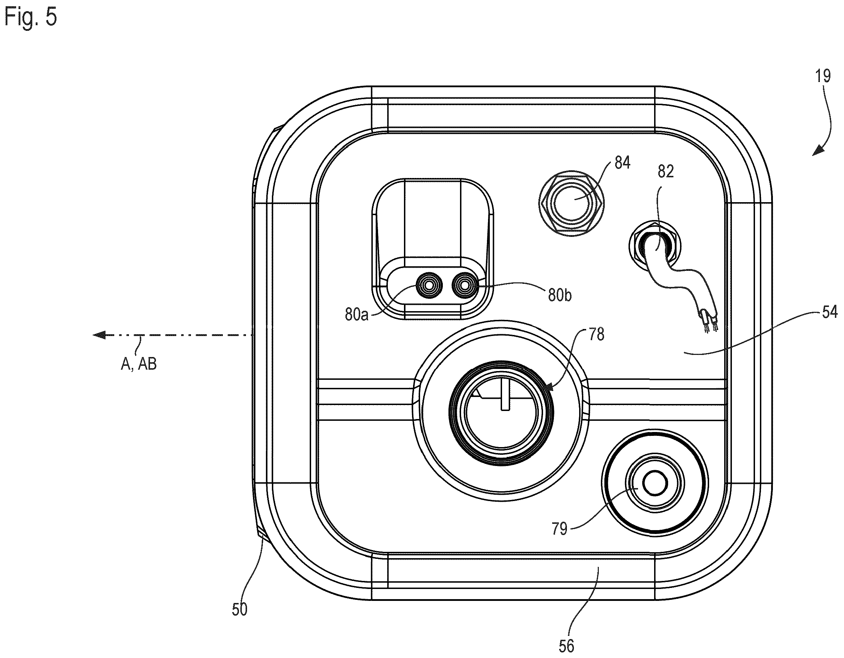

Lediglich der Vollständigkeit halber ist in Figur 5 ein Blick auf die Stirnseite 19 des Notfall-Beatmungsgeräts 10 dargestellt. Figur 5 zeigt eine Atemgas-Ausgabeöffnung 78, durch welche hindurch vom Gebläse der Druckveränderungsvorrichtung gefördertes inspiratorisches Atemgas aus dem Vorrichtungsgehäuse 12 zu einem an das Notfall-Beatmungsgerät 10 angeschlossenen Patienten hin austritt. Die Atemgas-Ausgabeöffnung 78 bildet ein Ende einer Atemgasleitung der Funktionsanordnung 60. An einem Sondergas-Kupplungsabschnitt 79 kann eine Liefervorrichtung zur Lieferung eines von der durch das Gebläse angesaugten Luft als Atemgas abweichenden Gases als inspiratorisches Atemgas oder Bestandteil desselben in die Atemgasleitung eingeleitet werden.

Figur 5 zeigt weiter Anschlussstutzen 80a und 80b, an welche Druckerfassungsschläuche anschließbar sind, die an ihrem von den Anschlussstutzen 80a bzw. 80b fernliegenden anderen Ende jeweils mit einem Innenbereich eines Differenzdruck-Flusssensors zur Messung eines proximalen inspiratorischen und bevorzugt auch exspiratorischen Atemgasstroms verbunden sind. Die beiden Innenbereiche des Differenzdruck-Flusssensors sind in an sich bekannter Weise durch einen durch den Atemgasstrom veränderlichen Strömungswiderstand voneinander getrennt.

Über einen Netzeingang 82 ist das Notfall-Beatmungsgerät 10 bei räumlicher Verfügbarkeit eines Netzanschlusses mit Energie aus einem externen Stromversorgungsnetz betreibbar, wie etwa aus einem öffentlichen Stromversorgungsnetz oder aus dem Bordversorgungsnetz eines Fahrzeugs oder Flugzeugs. Alle elektrischen Funktionseinheiten des Notfall-Beatmungsgeräts 10 können dann mit Netzenergie versorgt werden, wobei die Netzspannung über ein Netzteil als eine weitere Funktionseinheit der vormontierten Funktionsanordnung 60, auf eine Niedervolt-Gleichspannung transformiert wird. Ebenso kann der nicht dargestellte Akkumulator aufgeladen werden. Eine Buchse 84 im Gehäuse 12 ist zum Anschluss eines externen Sensors, insbesondere CO₂-Sensors, angeordnet. Ein solcher CO₂-Sensor kann beispielsweise an einem mit der Notfall-Beatmungsvorrichtung 10 gekoppelten Durchflusssensor vorgesehen und zu einer Sensoranordnung gekoppelt sein.

## Patentansprüche

1. Beatmungsvorrichtung (10) zur wenigstens unterstützenden künstlichen Beatmung von Patienten, umfassend:
- ein Vorrichtungsgehäuse (12),
- eine im Vorrichtungsgehäuse (12) in einer Betriebsposition aufgenommene Funktionsanordnung (60), welche als Funktionseinheiten wenigstens einen Abschnitt einer Atemgasleitung (78), eine Druckveränderungsvorrichtung zur Veränderung eines Atemgasdrucks in der Atemgasleitung (78) und eine Steuervorrichtung (62) zur Steuerung des Betriebs wenigstens der Druckveränderungsvorrichtung aufweist, und
- eine am Vorrichtungsgehäuse (12) in einer Anordnungsposition angeordnete, von außerhalb des Vorrichtungsgehäuses (12) zu ihrer Bedienung zugängliche Eingabe/Ausgabevorrichtung (40) zur Eingabe von Daten oder/und Steuerbefehlen an die Steuervorrichtung (62) oder/und zur Ausgabe von Daten und Information, wobei die Eingabe/Ausgabevorrichtung (40) durch eine Öffnung (14g) im Vorrichtungsgehäuse (12) hindurch signalübertragungsmäßig mit der Steuervorrichtung (62) verbunden ist,
wobei die Eingabe/Ausgabevorrichtung (40) unmittelbar an der Funktionsanordnung (60) gegen ein Entfernen der Eingabe/Ausgabevorrichtung (40) aus ihrer Anordnungsposition in einer Abheberichtung (A) vom Vorrichtungsgehäuse (12) weg gesichert ist,
wobei die Eingabe/Ausgabevorrichtung (40) durch eine an der Eingabe/Ausgabevorrichtung (40) ausgebildete oder angeordnete Verriegelungsformation (76) und eine an der Funktionsanordnung (60) ausgebildete oder angeordnete Verriegelungsgegenformation (66) an der Funktionsanordnung (60) gegen ein Entfernen aus ihrer Anordnungsposition in der Abheberichtung (A) vom Vorrichtungsgehäuse (12) weg gesichert ist,
**dadurch gekennzeichnet, dass** die Verriegelungsformation (76) und die Verriegelungsgegenformation (66) bei betriebsbereiter Beatmungsvorrichtung (10) in einem Verriegelungs-Formschlusseingriff miteinander stehen, wobei wenigstens eine Formation (66) aus Verriegelungsformation (76) und Verriegelungsgegenformation (76) wenigstens einmal aus einer Freigabestellung längs einer Bewegungsbahn (B) quer zu der Abheberichtung (A) in eine Verriegelungsstellung beweglich ist, wobei in der Freigabestellung kein Verriegelungs-Formschlusseingriff zwischen Verriegelungsformation (76) und Verriegelungsgegenformation (66) besteht und die dann unverriegelte Eingabe/Ausgabevorrichtung (40) aus ihrer Anordnungsposition in der Abheberichtung (A) von dem Vorrichtungsgehäuse (12) weg entfernbar ist, und wobei in der Verriegelungsstellung der Verriegelungs-Formschlusseingriff hergestellt ist.

2. Beatmungsvorrichtung (10) nach Anspruch 1,
wobei die Eingabe/Ausgabevorrichtung (40) nur an der Funktionsanordnung (60) gegen ein Entfernen aus ihrer Anordnungsposition in der Abheberichtung (A) vom Vorrichtungsgehäuse (12) weg gesichert ist.

3. Beatmungsvorrichtung (10) nach Anspruch 1 oder 2,
wobei die Eingabe/Ausgabevorrichtung (40) unter Zwischenanordnung eines Abschnitts des Vorrichtungsgehäuses zwischen einem Abschnitt der Eingabe/Ausgabevorrichtung (40) und einem Abschnitt der Funktionsanordnung (60) an der Funktionsanordnung (60) gegen ein Entfernen der Eingabe/Ausgabevorrichtung (40) aus ihrer Anordnungsposition in einer Abheberichtung (A) vom Vorrichtungsgehäuse (12) weg gesichert ist.

4. Beatmungsvorrichtung (10) nach Anspruch 3,
wobei die Eingabe/Ausgabevorrichtung (40) an der Funktionsanordnung (60) in Richtung auf das Vorrichtungsgehäuse zu gespannt ist.

5. Beatmungsvorrichtung (10) nach einem der vorhergehenden Ansprüche,
wobei die Eingabe/Ausgabevorrichtung (40) durch ein gesondert von der Eingabe/Ausgabevorrichtung (40) und der Funktionsanordnung (60) ausgebildetes Befestigungsmittel an der Funktionsanordnung (60) gegen ein Entfernen aus ihrer Anordnungsposition in der Abheberichtung (A) vom Vorrichtungsgehäuse (12) weg gesichert ist.

6. Beatmungsvorrichtung einem der vorhergehenden Ansprüche,
wobei das Vorrichtungsgehäuse (12) eine prismatische Gestalt mit einer mit radialem Abstand von einer Prismenachse (P) um die Prismenachse (P) umlaufenden Gehäusemantelwand (14) und mit wenigstens einer, bezogen auf die Prismenachse (P), axial endseitigen Öffnung (24, 25) aufweist.

7. Beatmungsvorrichtung (10) nach einem der vorhergehenden Ansprüche, sofern rückbezogen nach Anspruch 3,
wobei wenigstens eine Fläche (66a1, 66b1, 66c2, 76a1, 76b1, 76c1), vorzugsweise beide Flächen (66a1, 66b1, 66c2, 76a1, 76b1, 76c1), aus einer Anlagefläche (76a1, 76b1, 76c1) der Verriegelungsformation (76) und einer bei hergestelltem Verriegelungs-Formschlusseingriff an der Anlagefläche (76a1, 76b1, 76c1) anliegenden Gegenanlagefläche (66a1, 66b1, 66c2) der Verriegelungsgegenformation (66) derart zur Bewegungsbahn (B) geneigt sind, dass während einer Bewegung der wenigstens einen beweglichen Formation (66) aus Verriegelungsformation (76) und Verriegelungsgegenformation (66) von der Freigabestellung in die Verriegelungsstellung zur Herstellung des Verriegelungs-Formschlusseingriffs durch einen gleitenden Anlageeingriff zwischen Anlagefläche (76a1, 76b1, 76c1) und Gegenanlagefläche (66a1, 66b1, 66c2) eine Kraftwirkung auf die Eingabe/- Ausgabevorrichtung (40) in Richtung auf das Vorrichtungsgehäuse (12) zu ausgeübt wird.

8. Beatmungsvorrichtung (10) nach einem der vorhergehenden Ansprüche,
wobei nur die Verriegelungsgegenformation (66) zwischen der Freigabestellung und der Verriegelungsstellung bewegbar ist.

9. Beatmungsvorrichtung (10) nach einem der vorhergehenden Ansprüche,
wobei während einer Montage der Beatmungsvorrichtung (10) die Funktionsanordnung (60) durch eine Einführbewegung längs einer Einführbahn (E) in ihre Betriebsposition im Vorrichtungsgehäuse (12) einführbar ist und die Eingabe/Ausgabevorrichtung (40) durch eine Anordnungsbewegung längs einer Anordnungsbahn (AB) in ihre Anordnungsposition verlagerbar ist, wobei eine Anordnung (60) aus der Eingabe/Ausgabevorrichtung (40) und der Funktionsanordnung (60) einen Stecker (64) einer Signale oder/und Energie übertragenden Verbindungsleitung und die jeweils andere Anordnung (40) aus der Eingabe/Ausgabevorrichtung (40) und der Funktionsanordnung (60) eine Buchse (74) der Signale oder/und Energie übertragenden Verbindungsleitung aufweist, wobei der Stecker (64) und die Buchse (74) derart starr und längs der Einführbahn (E) oder der Anordnungsbahn (AB) aufeinander zuweisend ausgerichtet mit der sie jeweils aufweisenden Anordnung (40, 60) verbunden sind, dass eine zeitliche Abfolge einer Montagebewegung aus Einführbewegung und Anordnungsbewegung und der jeweils anderen Montagebewegung aus Einführbewegung und Anordnungsbewegung eine funktionstüchtige Verbindung des Steckers (64) mit der Buchse (74) bewirkt.

10. Beatmungsvorrichtung (10) nach einem der vorhergehenden Ansprüche,
wobei die Funktionsanordnung (60) eine vormontierte Baugruppe aus wenigstens dem Abschnitt einer Atemgasleitung (78), der Druckveränderungsvorrichtung und der Steuervorrichtung (62) ist, welche als vormontierte Baugruppe in das Vorrichtungsgehäuse (12) einführbar oder/und aus diesem entnehmbar ist.

## Claims

1. Ventilation device (10) for at least supporting artificial ventilation of patients, comprising:
- a device housing (12),
- a functional arrangement (60) accommodated in the device housing (12) in an operating position, which comprises as functional units at least one section of a breathing gas line (78), a pressure-changing device for changing a respiratory gas pressure in the respiratory gas line (78), and a control device (62) for controlling the operation of at least the pressure-changing device, and
- an input/output device (40) arranged on the device housing (12) in an arrangement position and accessible from outside the device housing (12) for its operation, for inputting data and/or control commands to the control device (62) and/or for outputting data and information, wherein the input/output device (40) is connected to the control device (62) in a signal transmitting fashion through an opening (14g) in the device housing (12),
wherein the input/output device (40) is secured directly to the functional arrangement (60) against removal of the input/output device (40) from its arrangement position in a lifting direction (A) away from the device housing (12),
**characterized in that** the input/output device (40) is secured to the functional arrangement (60) against removal from its assembly position in the lifting direction (A) away from the device housing (12) by a locking formation (76) formed or arranged on the input/output device (40) and a locking counter-formation (66) formed or arranged on the functional arrangement (60),
wherein the locking formation (76) and the locking counter-formation (66) are secured in a form-fitting engagement with each other when the ventilation device (10) is ready for operation, wherein at least one formation (66) of locking formation (76) and locking counter-formation (76) is movable at least once from a release position along a movement path (B) transverse to the lifting direction (A) into a locking position, wherein in the release position there is no locking form-fitting engagement between locking formation (76) and the locking counter-formation (66), and the then unlocked input/output device (40) can be removed from its arrangement position in the lifting direction (A) away from the device housing (12), and wherein in the locking position the locking form-fitting engagement is established.

2. Ventilation device (10) according to claim 1,
wherein the input/output device (40) is secured only at the functional arrangement (60) against removal from its arrangement position in the lifting direction (A) away from the device housing (12).

3. Ventilation device (10) according to claim 1 or 2,
wherein the input/output device (40) is secured to the functional arrangement (60) against removal of the input/output device (40) from its arrangement position in the lifting direction (A) away from the device housing (12) by interposition of a section of the device housing between a section of the input/ output device (40) and a section of the functional arrangement (60).

4. Ventilation device (10) according to claim 3,
wherein the input/output device (40) is biased towards the functional arrangement (60) in the direction of the device housing.

5. Ventilation device (10) according to one of the preceding claims,
wherein the input/ output device (40) is secured to the functional arrangement (60) against removal from its arrangement position in the lifting direction (A) away from the device housing (12) by a fastening means formed separately from the input/output device (40) and the functional arrangement (60).

6. Ventilation device according to one of the preceding claims,
wherein the device housing (12) has a prismatic shape with a housing shell wall (14) extending around a prism axis (P) at a radial distance from the prism axis (P) and with at least one axially, relative to the prism axis (P), end-side opening (24, 25).

7. Ventilation device (10) according to one of the preceding claims, insofar as referred back to claim 3,
wherein at least one surface (66a1, 66b1, 66c2, 76a1, 76b1, 76c1), preferably both surfaces (66a1, 66b1, 66c2, 76a1, 76b1, 76c1), out of an abutment surface (76a1, 76b1, 76c1) of the locking formation (76) and an abutment counter-surface (66a1, 66b1, 66c2) of the locking counter-formation (66) resting against the abutment counter-surface (76a1, 76b1, 76c1) when a positive locking form-fitting engagement is established, are inclined relative to the movement path (B) such that during movement of the at least one movable formation (66) out of locking formation (76) and locking counter-formation (66) from the release position to the locking position to establish the positive locking form-fitting engagement, a force effect is applied to the input/output device (40) in the direction towards the device housing (12) by a sliding abutting engagement between abutment surface (76a1, 76b1, 76c1) and abutment counter-surface (66a1, 66b1, 66c2).

8. Ventilation device (10) according to one of the preceding claims,
wherein only the locking counter-formation (66) is movable between the release position and the locking position.

9. Ventilation device (10) according to one of the preceding claims,
wherein, during assembly of the ventilation device (10), the functional arrangement (60) can be inserted into its operating position in the device housing (12) by an insertion movement along an insertion path (E), and the input/output device (40) can be displaced into its arrangement position by an arrangement movement along an arrangement path (AB), wherein an arrangement (60) out of the input/output device (40) and the functional arrangement (60) has a plug (64) of a connection line transmitting signals and/or energy, and the other arrangement (40) out of the input/output device (40) and the functional arrangement (60) has a socket (74) of the connection line transmitting signals and/or energy, wherein the plug (64) and the socket (74) are connected to the arrangement (40, 60), in which they are respectively provided, rigidly and aligned facing each other along the insertion path (E) or the arrangement path (AB), such that a temporal sequence of an assembly movement out of insertion movement and arrangement movement and the respective other assembly movement out of insertion movement and arrangement movement effects a functioning connection (40, 60) between the plug (64) and the socket (74).

10. Ventilation device (10) according to one of the preceding claims,
wherein the functional arrangement (60) is a preassembled assembly comprising at least the section of a breathing gas line (78), the pressure-changing device, and the control device (62), which can be inserted into and/or removed from the device housing (12) as a preassembled assembly.

## Revendications

1. Dispositif de ventilation (10) destiné à la ventilation artificielle au moins assistée de patients, comprenant :
- un boîtier du dispositif (12),
- un agencement fonctionnel (60) reçu dans le boîtier du dispositif (12) dans une position de fonctionnement, qui comprend, en tant qu'unités fonctionnelles, au moins une section d'une conduite de gaz respiratoire (78), un dispositif de modification de pression pour modifier une pression de gaz respiratoire dans la conduite de gaz respiratoire (78) et un dispositif de commande (62) pour commander le fonctionnement au moins du dispositif de modification de pression, et
- un dispositif d'entrée/sortie (40) disposé sur le boîtier (12) du dispositif dans une position d'agencement, accessible depuis l'extérieur du boîtier (12) du dispositif pour son utilisation, pour entrer des données et/ou des commandes dans le dispositif de commande (62) et/ou pour la sortie de données et d'informations, le dispositif d'entrée/sortie (40) étant connecté au dispositif de commande (62) par transmission de signaux à travers une ouverture (14g) dans le boîtier du dispositif (12),
le dispositif d'entrée/sortie (40) étant fixé directement sur l'agencement fonctionnel (60) afin d'empêcher le retrait du dispositif d'entrée/sortie (40) de sa position d'agencement dans une direction de soulèvement (A) à partir du boîtier du dispositif (12),
**caractérisé en ce que** le dispositif d'entrée/sortie (40) est fixé à l'agencement fonctionnel (60) par une formation de verrouillage (76) formée ou disposée sur le dispositif d'entrée/sortie (40) et une contre-formation de verrouillage (66) formée ou disposée sur l'agencement fonctionnel (60) contre un retrait de sa position d'agencement dans la direction de soulèvement (A) à partir du boîtier (12) du dispositif,
la formation de verrouillage (76) et la contre-formation de verrouillage (66) étant en engagement de verrouillage par complémentarité de forme lorsque le dispositif de ventilation (10) est prêt à fonctionner, au moins une formation (66) sélectionnée d'une formation de verrouillage (76) et d'une contre-formation de verrouillage (76) étant mobile au moins une fois d'une position de libération le long d'une trajectoire de mouvement (B) transversale à la direction de soulèvement (A) vers une position de verrouillage, aucune engagement de verrouillage par complémentarité de forme n'existant entre la formation de verrouillage (76) et la contre-formation de verrouillage (66) dans la position de libération et le dispositif d'entrée/sortie (40) alors déverrouillé peut être retiré de sa position d'agencement dans la direction de soulèvement (A) du boîtier du dispositif (12), et dans lequel, dans la position de verrouillage, l'engagement de verrouillage par complémentarité de forme est établi.

2. Dispositif de ventilation (10) selon la revendication 1,
dans lequel le dispositif d'entrée/sortie (40) est uniquement fixé à l'agencement fonctionnel (60) pour empêcher son retrait de sa position d'agencement dans la direction de soulèvement (A) du boîtier du dispositif (12).

3. Dispositif de ventilation (10) selon la revendication 1 ou 2,
dans lequel le dispositif d'entrée/sortie (40) est fixé à l'agencement fonctionnel (60) contre un retrait du dispositif d'entrée/sortie (40) de sa position d'ensemble dans la direction de soulèvement (A) à l'aide d'une partie du boîtier du dispositif (12) intercalée entre une partie du dispositif d'entrée/sortie (40).

4. Dispositif de ventilation (10) selon la revendication 3,
dans lequel le dispositif d'entrée/sortie (40) est serré sur l'agencement fonctionnel (60) en direction du boîtier du dispositif.

5. Dispositif de ventilation (10) selon l'une des revendications précédentes,
dans lequel le dispositif d'entrée/sortie (40) est fixé à l'agencement fonctionnel (60) par un moyen de fixation formé séparément du dispositif d'entrée/sortie (40) et de l'agencement fonctionnel (60) afin d'empêcher son retrait de sa position d'agencement dans la direction de soulèvement (A) à partir du boîtier (12) du dispositif.

6. Dispositif de ventilation selon l'une des revendications précédentes,
le boîtier (12) du dispositif présentant une forme prismatique avec une paroi d'enveloppe (14) s'étendant autour de l'axe (P) du prisme à une distance radiale de celui-ci et avec au moins une ouverture (24, 25) à l'extrémité axiale rapportée à l'axe (P) du prisme.

7. Dispositif de ventilation (10) selon l'une quelconque des revendications précédentes, à condition qu'il soit rétroactif selon la revendication 3,
dans lequel au moins une surface (66a1, 66b1, 66c2, 76a1, 76b1, 76c1), de préférence les deux surfaces (66a1, 66b1, 66c2, 76a1, 76b1, 76c1), sélectionnée d'une surface d'appui (76a1, 76b1, 76c1) de la formation de verrouillage (76) et une contre-surface d'appui (66a1, 66b1, 66c2) de la contre-formation de verrouillage (66) s'appliquant contre la surface d'appui (76a1, 76b1, 76c1) lorsque l'engagement de verrouillage par complémentarité de forme est établi., sont inclinées par rapport à la trajectoire de déplacement (B) de telle sorte, que, pendant un mouvement de la au moins une formation mobile (66) de la formation de verrouillage (76) et de la contre-formation de verrouillage (66) de la position de libération à la position de verrouillage pour établir l'engagement de verrouillage de la forme, un engagement d'appui glissant entre la surface d'appui (76a1, 76b1, 76c1) et la contre-surface d'appui (66a1, 66b1, 66c2) exerce une action de force sur le dispositif d'entrée/sortie (40) en direction du boîtier du dispositif (12).

8. Dispositif de ventilation (10) selon l'une des revendications précédentes,
dans lequel seule la contre-formation de verrouillage (66) est mobile entre la position de déverrouillage et la position de verrouillage.

9. Dispositif de ventilation (10) selon l'une des revendications précédentes,
dans lequel, pendant le montage du dispositif de ventilation (10), l'agencement fonctionnel (60) peut être introduit dans sa position de fonctionnement dans le boîtier du dispositif (12) par un mouvement d'insertion le long d'une trajectoire d'insertion (E) et le dispositif d'entrée/sortie (40) peut être déplacé dans sa position d'agencement par un mouvement de disposition le long d'une trajectoire de disposition (AB), un agencement (60) sélectionné du dispositif d'entrée/sortie (40) et de l'agencement fonctionnel (60) comportant un fiche (64) d'un câble de connexion transmettant des signaux et/ou de l'énergie et l'autre agencement (40) sélectionné du dispositif d'entrée/sortie (40) et de l'agencement fonctionnel (60) comportant une prise (74) du câble de raccordement transmettant des signaux et/ou de l'énergie, la fiche (64) et la prise (74) étant connectés de manière rigide et orientées le long de la trajectoire d'introduction (E) ou de la trajectoire d'agencement (AB) l'un vers l'autre à l'agencement (40,60) qui les présente respectivement, de telle sorte qu'une succession temporelle d'un mouvement de montage sélectionné d'un mouvement d'introduction et d'un mouvement d'agencement et de l'autre mouvement de montage sélectionné d'un mouvement d'introduction et d'un mouvement d'agencement provoque une connexion fonctionnelle de la fiche (64) avec la prise (74).

10. Dispositif de ventilation (10) selon l'une des revendications précédentes, l'agencement fonctionnel (60) étant un ensemble prémonté composé au moins de la section d'une conduite de gaz respiratoire (78), du dispositif de modification de pression et du dispositif de commande (62), lequel ensemble prémonté peut être inséré dans le boîtier du dispositif (12) et/ou retiré de celui-ci.
